# FASCICULE DE BREVET EUROPEEN

(11) **EP 2 705 873 B1**
(45) Date de publication et mention de la délivrance du brevet: **15.02.2017**
(21) Numéro de dépôt: 13183781.7
(22) Date de dépôt: 10.09.2013
(51) Int. Cl.: A61M 39/12, A61M 39/28, A61M 39/24, A61M 39/26

(54) **Système de blocage amovible d'un cathéter**
Entfernbares Blockiersystem eines Katheters
System for removable locking of a catheter

(30) Priorité: 11.09.2012 FR 1258528
(43) Date de publication de la demande: 12.03.2014
(73) Titulaire: Vygon, 95440 Ecouen (FR)
(72) Inventeur: Dulong, Claire, 60126 Rivecourt (FR); Carrez, Jean-Luc, 95440 Ecouen (FR); Guyomarc'h, Pierrick, 95120 Ermont (FR); Coussegal, Jean-Louis, 95250 Beauchamp (FR)
(74) Mandataire: Regimbeau

(56) Documents cités:
- WO-A1-2010/028021
- WO-A2-00/03165
- US-A- 3 856 010
- US-A1- 2003 225 379
- US-A1- 2010 274 174

## Description

### Domaine de l'invention

L'invention concerne de manière générale un système de blocage amovible adapté pour mettre en communication fluidique un dispositif médical et un cathéter.

### Arrière-plan de l'invention

Les cathéters, et en particulier les cathéters d'anesthésie locorégionale, sont de petit diamètre, le plus souvent de l'ordre de 0.43*0.80 mm, 0.50*0.90 mm ou encore 0.50*1.00 mm, et réalisés dans un matériau relativement élastique afin d'éviter notamment les problèmes de plicature. Etant donnée la faible dimension de ces cathéters, ils sont donc difficiles à bloquer de manière ergonomique dans une embase. Ceci est cependant nécessaire si l'on souhaite connecter le cathéter à un dispositif médical, tel qu'un filtre dans le cadre des anesthésies locorégionales.

Il a donc été proposé de mettre en oeuvre une embase, comprenant un corps adapté pour loger un manchon en matériau élastomère, présentant une lumière interne destinée à recevoir le cathéter, et pouvant être comprimé à l'aide d'un écrou par vissage. La compression radiale du manchon par l'écrou permet en effet de maintenir le cathéter en position dans l'embase. Ce type d'embase est par exemple illustré dans la demande de brevet au nom de la Demanderesse n°FR 2 896 698.

A l'usage, la Demanderesse a constaté que l'écrou permettant de comprimer le manchon risquait d'être perdu, notamment au moment où celui-ci est dévissé afin de permettre l'introduction du cathéter dans le manchon compressible. Il n'est alors plus possible d'utiliser l'embase, ou du moins faut-il remplacer la pièce perdue. Par ailleurs, lors de son vissage, l'écrou risque d'entrainer le manchon compressible en rotation en le tordant sur lui-même, de sorte qu'une fois le cathéter bloqué, le manchon risque de reprendre sa forme initiale en entrainant à son tour l'écrou, relâchant ainsi la contrainte appliquée au cathéter, ce qui peut être nuisible à une bonne tenue mécanique du cathéter dans l'embase.

De plus, lorsque l'embase est fixée à un dispositif médical tel qu'un filtre et que celui-ci doit être changé ou simplement retiré, le dévissage du dispositif médical de l'embout (généralement de type normalisé Luer ou Luer Lock) de l'embase peut entrainer le dévissage de l'écrou, dans la mesure où il est nécessaire de tenir l'embase par l'embout lors du dévissage du dispositif médical.

La Demanderesse a également constaté qu'il était difficile de déterminer si le cathéter était correctement maintenu dans l'embase, dans la mesure où rien ne permet de confirmer que l'écrou est suffisamment vissé sur le manchon compressible ou que le cathéter est correctement inséré dans l'embase. De plus, le vissage n'est aujourd'hui pas considéré comme ergonomique.

Il a également été proposé d'utiliser une embase comprenant un manchon tubulaire relativement mince, présentant une lumière centrale dont le diamètre est sensiblement égal à celui du cathéter devant être bloqué, et destiné à être comprimé radialement.

Par exemple, le document WO 2011/106077 propose un système comprenant une embase, comportant un manchon mince déformable adapté pour recevoir un cathéter et une coque munie de nervures internes. La coque est montée à rotation sur l'embase et adaptée pour déformer radialement le manchon à l'aide des nervures internes lorsqu'elle est fermée sur l'embase, de manière à réduire localement le diamètre interne du manchon et à bloquer le cathéter en position.

Le document US 2003/0225379 quant à lui décrit une valve adaptée pour recevoir de manière amovible un cathéter et empêcher, lorsque nécessaire, le passage de fluides dans ce cathéter. A cet effet, la valve comprend une embase adaptée pour recevoir le cathéter, et une coque, montée en translation sur l'embase entre une position ouverte, dans laquelle la coque est éloignée de l'embase, et une position fermée, dans laquelle la coque est accrochée sur l'embase et bloque le cathéter en position. L'embase définit un logement abritant un manchon pourvu d'une lumière de blocage adaptée pour recevoir le cathéter. Le système comprend en outre un actionneur mobile en translation dans le logement entre une position de repos et une position de blocage dans laquelle l'actionneur applique un effort transversal sur le manchon afin de le déformer localement. Dans cette position de blocage ponctuelle, l'actionneur déforme également le cathéter afin d'empêcher le passage de fluides.

### Résumé de l'invention

Un objectif de l'invention est de proposer un système de blocage amovible d'un cathéter dans une embase, qui soit ergonomique, facile à mettre en oeuvre et efficace, sans nécessiter d'efforts trop importants de la part d'un opérateur.

De manière optionnelle, un objectif est également de garantir à un opérateur que le cathéter est bien bloqué par le système, d'éviter que des pièces puissent être facilement égarées et que le système reste verrouillé, sauf intervention d'un opérateur.

A cet effet, l'invention propose un système de blocage amovible d'un cathéter, notamment un cathéter d'anesthésie locorégionale, comprenant une embase adaptée pour recevoir un cathéter, et une coque, montée à rotation sur l'embase entre une position ouverte, dans laquelle la coque est éloignée de l'embase, et une position fermée, dans laquelle la coque est accrochée sur l'embase et bloque le cathéter en position, le système étant caractérisé en ce que :
- l'embase définit un logement présentant une direction principale selon un axe longitudinal, ledit logement abritant un manchon pourvu d'une lumière de blocage s'étendant le long de l'axe longitudinal et adapté pour venir en butée contre une paroi distale du logement,
- le système comprend un actionneur pourvu d'une lumière de guidage s'étendant le long de l'axe longitudinal et mobile en translation le long de cet axe longitudinal dans le logement entre une position de repos et une position de blocage dans laquelle l'actionneur applique un effort axial sur le manchon afin de le déformer, l'actionneur comprenant un premier organe d'entraînement,

- la lumière de blocage et la lumière de guidage sont adaptées pour recevoir un cathéter, et
- la coque comprend un deuxième organe d'entraînement, adapté pour coopérer avec le premier organe d'entraînement de l'actionneur lorsque la coque est déplacée de sa position ouverte vers sa position fermée, et déplacer l'actionneur le long de l'axe longitudinal vers sa position de blocage.

Certaines caractéristiques optionnelles mais non limitatives d'un système de blocage amovible conforme à l'invention sont les suivantes :
- le logement comprend :
   * une chambre de blocage,
   * une bague, s'étendant dans le prolongement de la chambre de blocage, le long de l'axe longitudinal, et
   * un canal, s'étendant entre la chambre de blocage et la bague, le long de l'axe longitudinal,
- un diamètre externe de la partie distale de l'actionneur est au plus égal au diamètre interne de la bague,
- un diamètre interne de la lumière de blocage du manchon est sensiblement égal au diamètre externe du cathéter,
- le logement comprend une ouverture s'étendant en regard du premier organe d'entraînement de l'actionneur, le deuxième organe d'entraînement de la coque étant positionné de manière à coopérer avec le premier organe d'entraînement à travers ladite ouverture du logement,
- le premier organe d'entraînement possède une rampe de renvoi s'étendant vers l'extérieur depuis la partie distale de l'actionneur, et le deuxième organe d'entraînement comprend une partie en saillie s'étendant depuis la coque, agencée de manière à venir en contact avec ladite rampe de renvoi lorsque la coque est amenée vers sa position fermée,
- l'organe est de forme globalement annulaire, et la rampe de renvoi est orientée en oblique par rapport à un plan normal à la direction longitudinale,
- l'organe comprend en outre une paroi proximale de verrouillage s'étendant depuis la rampe de renvoi dans un plan sensiblement perpendiculaire à la direction longitudinale, adaptée pour venir en contact avec une paroi distale de la saillie lorsque la coque dans sa position fermée,
- la partie distale comprend en outre une protubérance s'étendant radialement depuis ladite partie distale, adaptée pour empêcher l'actionneur de sortir du logement,
- l'actionneur et le logement sont munis d'une ou plusieurs nervures et gorges rectilignes, s'étendant radialement depuis l'actionneur, adaptées pour empêcher la rotation de l'actionneur dans l'embase,
- l'embase comprend en outre un organe de connexion à un dispositif médical, ledit organe de connexion étant en communication fluidique avec le logement,
- le système comprend en outre une chambre transparente de visualisation s'étendant entre le logement et l'organe de connexion, adaptée pour recevoir une extrémité libre du cathéter,
- la coque comprend des moyens d'accrochage à l'embase,
- les moyens d'accrochage comprennent au moins un crochet engagé dans un orifice dans l'embase de manière à pouvoir être libéré pour ouvrir le système de blocage amovible, et
- les moyens d'accrochage comprennent des crochets, fixés sur la coque et adaptés pour coopérer par encliquetage avec des languettes s'étendant depuis l'embase, lesdites languettes étant élastiquement déformables de manière à permettre à un opérateur de libérer les crochets par simple pression.

### Brève description des dessins

D'autres caractéristiques, buts et avantages de la présente invention apparaîtront mieux à la lecture de la description détaillée qui va suivre, faite en référence aux figures annexées données à titre d'exemples non limitatifs et sur lesquelles :

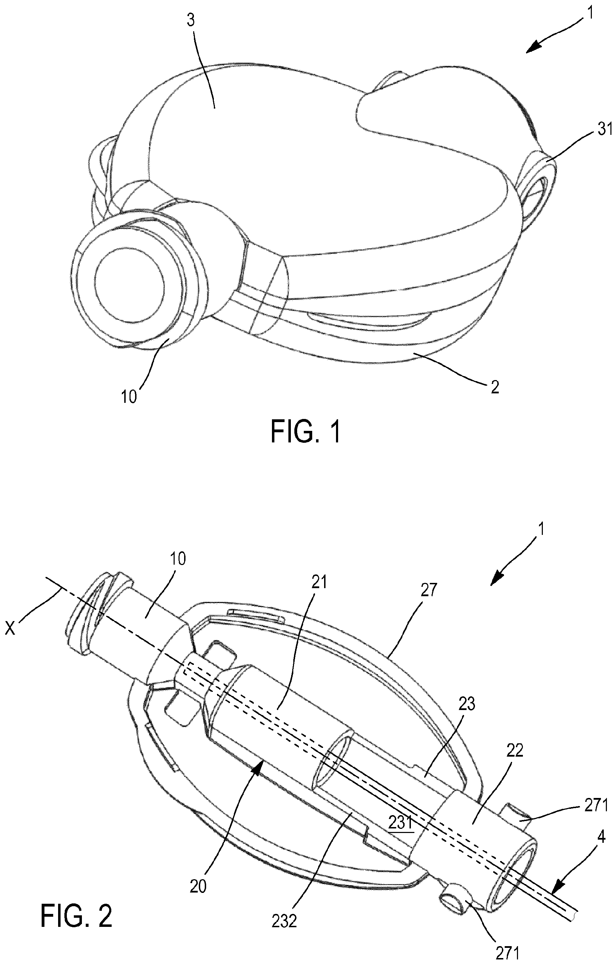

La figure 1 est une vue en perspective d'un exemple de réalisation d'un système de blocage amovible conforme à l'invention, en position fermée,
La figure 2 est une vue du dessus d'un exemple de réalisation d'une embase d'un système de blocage amovible conforme à l'invention, dans laquelle le manchon et l'actionneur n'ont pas été représentés,

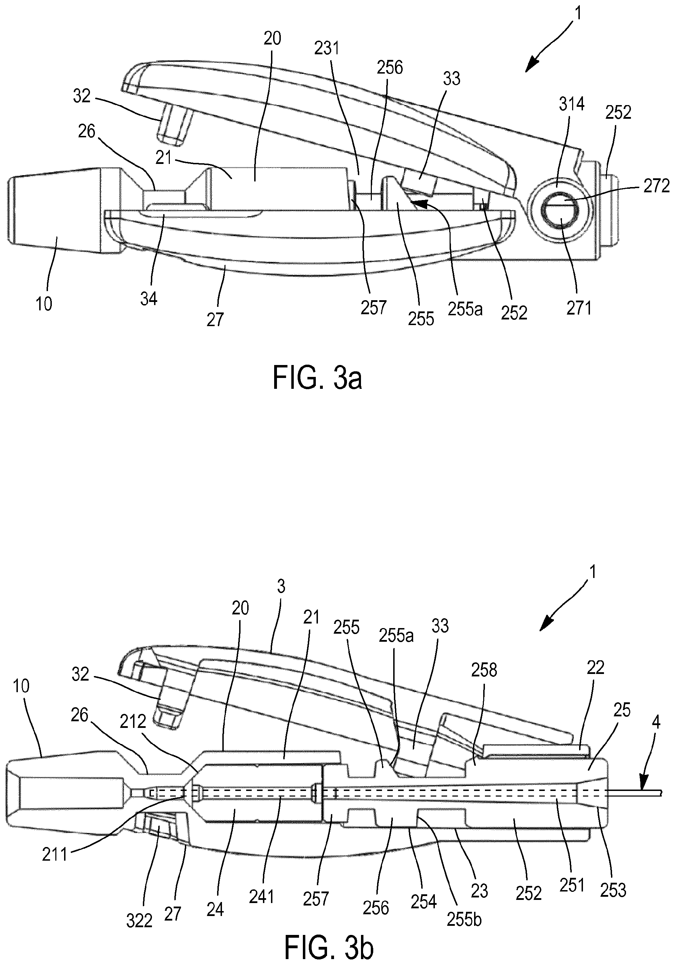

La figure 3a est une vue de côté du système de blocage amovible de la figure 1, le système étant ouvert,
La figure 3b est une vue en coupe transversale du système de blocage amovible de la figure 3a,

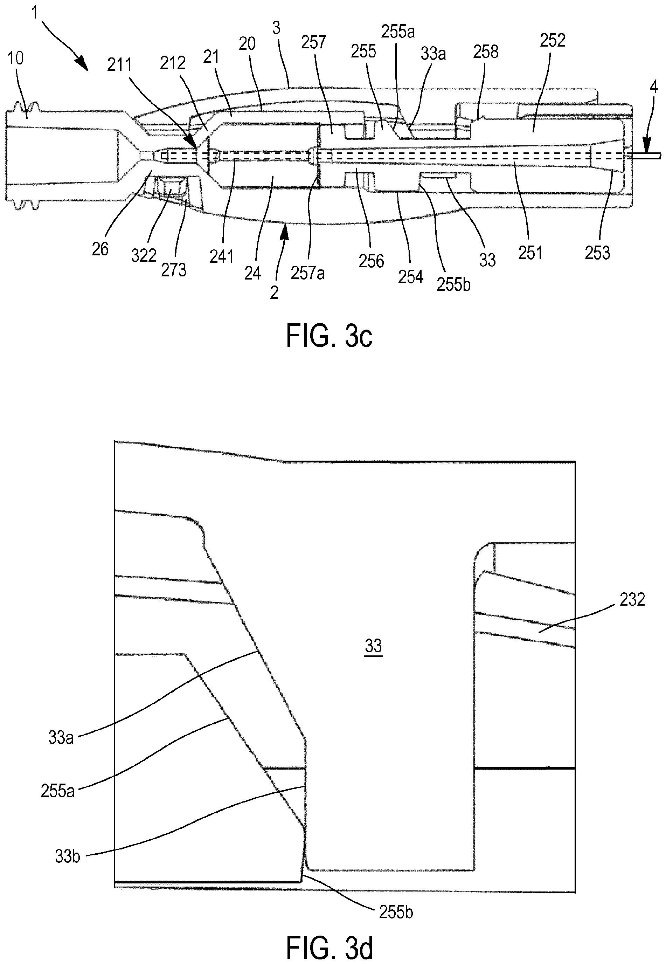

La figure 3c est une vue en coupe transversale du système de blocage amovible de la figure 3a, le système étant fermé,
La figure 3d est une vue de côté en détail d'un exemple de réalisation des moyens de blocage lorsque le système de blocage est en position fermée,
La figure 4 est une vue en coupe d'un exemple de réalisation de l'embase de la figure 2,
La figure 5a est une vue en coupe transversale selon un plan passant par l'axe longitudinal X de l'embase de la figure 2, dans laquelle des exemples de réalisation d'un manchon et d'un actionneur ont été représentés,
La figure 5b est une vue en perspective de l'embase munie du manchon et de l'actionneur de la figure 5a,
La figure 5c est une vue en coupe transversale en perspective, selon un plan parallèle au plan de coupe de la figure 5a, de l'embase munie du manchon et de l'actionneur de la figure 5a,
La figure 6 est une vue du dessous d'un exemple de réalisation d'un système de blocage amovible conforme à l'invention,
La figure 7 est une vue en perspective d'un exemple de réalisation d'un système de blocage amovible conforme à l'invention, en position ouverte,
La figure 8a est une vue en perspective d'un autre exemple de réalisation d'un système de blocage amovible conforme à l'invention, en position ouverte,
La figure 8b est une vue en perspective de l'exemple de réalisation de la figure 8a, en position fermée, et
La figure 8c est une vue en perspective de la coque du système de blocage de la figure 8a, et
La figure 8d est une vue du dessus de l'embase du système de blocage de la figure 8a.

### Description détaillée d'une forme de réalisation

Un système de blocage amovible 1 d'un cathéter conforme à l'invention va à présent être décrit en référence aux figures annexées.

Le système comprend une embase 2, adaptée pour recevoir un cathéter 4, par exemple un cathéter d'anesthésie locorégionale, et une coque 3, pouvant être fixée sur l'embase 2 de manière à bloquer celui-ci en position de manière amovible. Le système de blocage 1 comprend en outre un organe de connexion 10 avec un dispositif médical, par exemple un filtre, permettant de mettre en communication fluidique le cathéter 4 (une fois celui-ci inséré et bloqué dans le système) avec le dispositif médical. Il peut s'agir de manière conventionnelle d'un embout du type Luer ou Luer Lock, mâle ou femelle.

La coque 3 comprend des moyens de fixation 31 à l'embase 2, de préférence par liaison à rotule, des moyens d'accrochage 32 à l'embase 2, ainsi qu'un organe d'entraînement 33.

L'embase 2 quant à elle comprend un logement 20, présentant une direction principale selon un axe longitudinal X, ledit logement 20 comprenant :
- une ouverture 231 disposée en regard de la coque 3,
- un manchon 24, logé dans le logement 20,
- un actionneur 25, mobile en translation dans le logement 20 entre une position de blocage, dans laquelle l'actionneur 25 applique un effort axial sur le manchon 24 afin de le déformer, et une position de repos, dans laquelle l'actionneur 25 n'applique pas d'effort substantiel sur le manchon 24, l'actionneur 25 comprenant un organe d'entraînement 255 adapté pour coopérer avec l'organe d'entraînement 33 de la coque 3, disposé au moins partiellement en regard de l'ouverture du logement 20, et
- une lumière 251, 241 adaptée pour recevoir un cathéter 4, traversant l'actionneur 25 et le manchon, et s'étendant le long de l'axe longitudinal X.

Par la suite, « proximal » désignera une partie (par exemple une extrémité) qui se situe à proximité d'un opérateur, tandis que « distal » désignera une partie qui est éloignée de l'opérateur.

Par exemple, le logement 20 comprend :
- une chambre de blocage 21,
- une bague 22, s'étendant dans le prolongement de la chambre de blocage 21, et
- un canal 23, s'étendant entre la chambre de blocage 21 et la bague 22, formant une ouverture 23.

La chambre de blocage 21, le canal 23 et la bague 22 s'étendent successivement selon l'axe longitudinal X, le long de la direction principale du logement 20. Ils peuvent être positionnés l'un par rapport à l'autre par fixation sur une base 27. En variante, le logement 20 peut être monobloc, puis fixé sur la base 27. Selon une autre variante encore, le logement 20 et la base 27 sont monoblocs.

Le manchon 24 est alors logé dans la chambre de blocage 21, tandis que l'actionneur 25 est logé au moins partiellement dans le canal 23 et la bague 22.

La coque 3 est de préférence articulée sur l'embase 2 au moyen d'une liaison à pivot. Pour cela, la base 27 peut comprendre par exemple deux picots 271, adaptés pour coopérer avec deux oreilles 311 formées d'orifices complémentaires s'étendant depuis la coque 3, au niveau d'une extrémité du système 1. Les picots 271 peuvent présenter un chanfrein 272 afin de faciliter le montage des oreilles 311 de la coque 3.

La chambre de blocage 21 peut par exemple présenter une forme globalement tubulaire, présentant une ouverture proximale débouchant dans le canal 23, et une ouverture distale 211 en communication fluidique avec l'organe de connexion 10 à un dispositif médical. L'ouverture distale 211 est réalisée au niveau d'une paroi distale 212 de la chambre de blocage 21, de forme généralement tronconique ou annulaire. Cette paroi distale 212 est adaptée pour recevoir le manchon 24 et le bloquer en translation, sans boucher l'ouverture distale 211.

L'embase 2 peut en outre comprendre une chambre transparente de visualisation 26, s'étendant entre la chambre de blocage 21 et l'organe de connexion 10. Cette chambre de visualisation 26 est alors réalisée dans un matériau transparent ou peut comprendre une fenêtre de visualisation réalisée dans un matériau transparent, afin de permettre à un opérateur de voir le contenu de la chambre de visualisation 26. La chambre de visualisation 26 est connectée à la chambre de blocage 21 par l'intermédiaire de la paroi distale 212. Elle peut notamment avoir une forme tubulaire, dont les dimensions correspondent sensiblement au diamètre de l'ouverture distale 211 de la chambre de blocage 21.

Comme on le verra dans la suite, la chambre de visualisation 26 est adaptée pour recevoir une extrémité libre du cathéter 4 et jouer le rôle de repère visuel pour le positionnement du cathéter 4 dans l'embase 2.

La chambre de visualisation 26 peut également permettre la fixation de la coque 3 sur l'embase 2 afin de fermer le système de blocage amovible 1. Les moyens d'accrochage 32 de la coque 3 sont alors prévus en regard de la chambre de visualisation 26. Notamment, les moyens de d'accrochage 32 peuvent comprendre deux pattes 321 adaptées pour se positionner de part et d'autre de la chambre 26 lorsque la coque 3 est rabattue sur l'embase 2, et présentant au niveau de leur extrémité libre des crochets 322 en regard adaptés pour venir en prise avec la chambre de visualisation 26. Les pattes 321 sont prévues pour s'écarter afin de glisser sur les parois latérales de la chambre de visualisation 26 jusqu'à ce que les crochets 322 viennent se loger sous la chambre de visualisation 26, où ils sont maintenus en position sous l'effet du retour élastique des pattes, maintenant ainsi la coque 3 fixée sur l'embase 2. De manière optionnelle, ce retour élastique peut notamment servir de repère auditif à l'opérateur afin de vérifier que le système 1 est correctement fermé.

Afin d'ouvrir le système 1, il suffit de déloger les crochets 322, de manière à pouvoir séparer la coque 3 de l'embase 2. Pour cela, un orifice 273 peut être réalisé dans une surface inférieure 274 de la base 27 de l'embase 2, en regard de la chambre de visualisation 26, afin de permettre le passage de l'embout d'un objet, tel qu'un embout de seringue, tandis que les crochets 322 peuvent présenter une surface inférieure 323 inclinée jouant le rôle de rampe. Il suffit alors à l'opérateur d'introduire l'objet dans l'orifice 273 de manière à appuyer sur les surfaces inférieures 323 des crochets 322, afin d'écarter les crochets 322 et de libérer la chambre de visualisation 26.

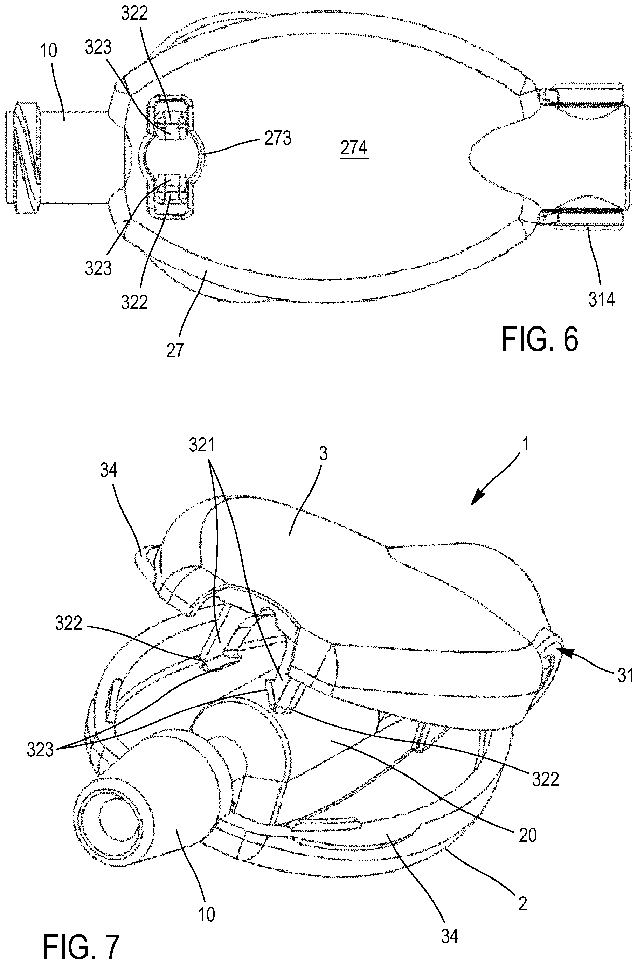

L'orifice 273 peut être dimensionné de manière à laisser le passage à l'embout de l'objet seul. En variante, les crochets 322 peuvent affleurer au niveau de la surface inférieure 274 de la base au travers de l'orifice 273, comme illustré sur la figure 6. L'orifice 273 est alors dimensionné de manière à autoriser l'introduction de l'embout de l'objet ainsi que l'écartement des crochets 322.

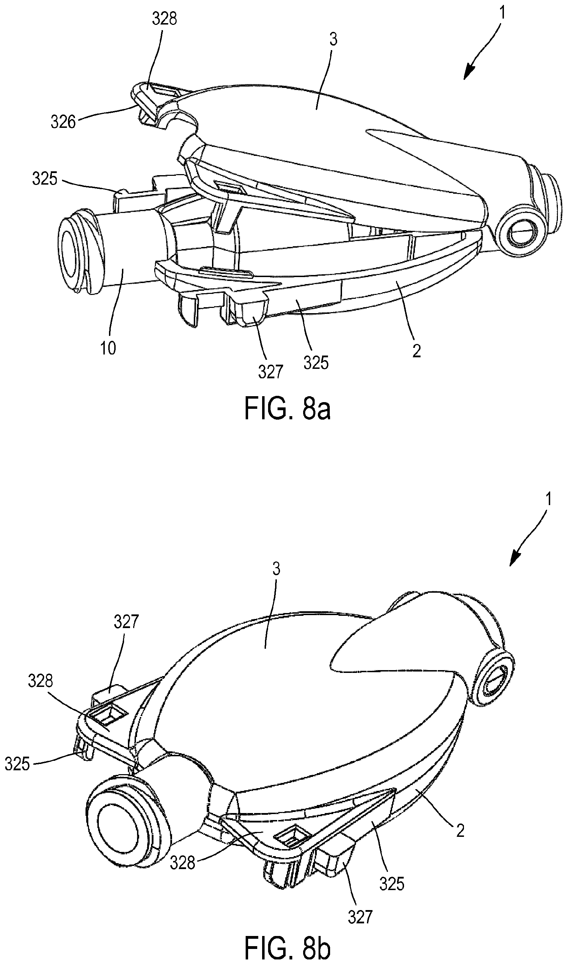

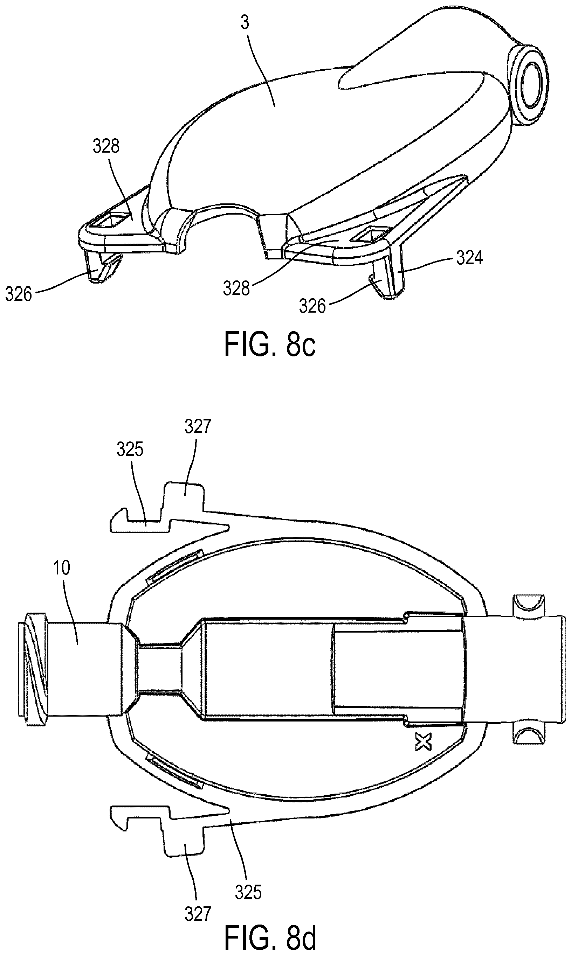

En variante, comme illustré sur les figures 8a à 8d, les moyens d'accrochage 32 de la coque 3 peuvent être prévus de part et d'autre de la coque 3 et de l'embase 2. Notamment, les moyens de d'accrochage 32 peuvent comprendre deux pattes latérales 324 s'étendant en direction de l'embase 2 lorsque la coque 3 est rabattue sur l'embase 2, et présentant au niveau de leur extrémité libre des crochets 326 en regard adaptés pour venir en prise avec des languettes d'encliquetage 325, s'étendant depuis l'embase 2.

Les languettes d'encliquetage 325 de l'embase 2 sont fixées sur l'embase 2, le long de ses côtés latéraux, et s'étendent sensiblement parallèlement à l'axe longitudinal X en direction du connecteur 10. De préférence, les languettes d'encliquetage 325 sont dimensionnées de manière à ne pas dépasser de la surface supérieure de l'embase 2. Les languettes d'encliquetage 325 sont déformables élastiquement, de manière à permettre le passage des crochets 326 et de les maintenir en position. Ainsi, les pattes latérales 324 sont prévues pour s'écarter afin de glisser sur les languettes d'encliquetage 325 jusqu'à ce que les crochets 326 viennent se loger sous les languettes d'encliquetage 325 où ils sont maintenus en position sous l'effet du retour élastique des languettes d'encliquetage 325, maintenant ainsi la coque 3 fixée sur l'embase 2. De manière optionnelle, ce retour élastique peut notamment servir de repère auditif à l'opérateur afin de vérifier que le système 1 est correctement fermé.

Dans la forme de réalisation illustrée sur les figures, les pattes latérales 324 sont fixées sur une surface 328 s'étendant dans le plan de la surface inférieure d la coque 3 de manière à être décalées par rapport aux côtés externes de ladite coque 3. De la sorte, lors de la fermeture de la coque 3 sur l'embase 2, les crochets 326 viennent en contact avec la surface externe des languettes d'encliquetage 325, afin de les déformer en les rapprochant de l'embase 2 jusqu'au passage desdits crochets 326.

Les languettes d'encliquetage 325 sont en outre chacune munies d'une protubérance 327, formées sur leur surface externe de manière à être accessibles par l'opérateur, afin de permettre à l'opérateur de déformer les pattes d'encliquetage 325 par simple pression des doigts en les rapprochant de l'embase 2, et donc de libérer les crochets 326. Par un mouvement volontaire, l'opérateur peut alors séparer la coque 3 de l'embase 2 et ouvrir le système de blocage amovible 1 afin de libérer le cathéter.

Avantageusement, l'encliquetage des pattes 324 et 325 constitue un repère auditif pour l'opérateur, lui permettant de s'assurer de la bonne fermeture du système de blocage amovible. Par ailleurs, les moyens d'accrochage 32 assurent une sécurité d'encliquetage, nécessitant une intervention volontaire de l'opérateur pour sa réouverture.

De manière optionnelle, la coque 3 et/ou l'embase 2 peuvent être munies de reliefs 34, par exemple des oreilles, afin de faciliter la manipulation du système 1 pour soulever la coque 3.

Le manchon 24 est réalisé de préférence dans un matériau déformable, pouvant être compressible. Par exemple, le manchon 24 peut être réalisé en matériau élastomérique, tel que du polyisoprène.

Le manchon 24 comprend une lumière de blocage 241 traversante destinée à recevoir le cathéter 4. Le diamètre interne de cette lumière de blocage 241 est choisi de manière à être sensiblement égal à celui du cathéter 4, afin de permettre son introduction dans la lumière 241 et faciliter ensuite son blocage lorsque le manchon 24 est déformé par l'actionneur 25. La lumière de blocage 241 est de préférence coaxiale au manchon 24.

La forme du manchon 24 est globalement complémentaire de la forme de l'espace interne de la chambre de blocage 21 afin de garantir son positionnement dans la chambre de blocage 21 et donc le positionnement de la lumière de blocage 241 qui est destinée à bloquer le cathéter 4. Ainsi, pour une chambre de blocage 21 de forme globalement tubulaire, le manchon 24 est de forme globalement cylindrique de révolution. Son diamètre est par ailleurs choisi en fonction du diamètre interne de la chambre de blocage 21, afin de permettre son insertion dans la chambre de blocage 21 et de garantir son positionnement par rapport au logement 20. Par exemple, le diamètre externe du manchon 24 peut être sensiblement égal au diamètre interne de la chambre de blocage 21.

Ainsi, une fois le manchon 24 positionné dans la chambre de blocage 21, la lumière de blocage 241 est coaxiale à la chambre de blocage 21 (et au logement 20).

La bague 22 peut également présenter une forme globalement tubulaire, et comprendre une ouverture proximale et une ouverture distale débouchant dans le canal 23. Dans la forme de réalisation illustrée sur les figures, la bague 22 porte les moyens de connexion à la coque 3, ici les deux picots 271 s'étendant radialement par rapport à l'axe longitudinal X, dans le plan de l'embase 2. La coque 3 comprend alors les deux oreilles 311, adaptées pour être montées sur les picots 271, permettant à la coque 3 de pivoter autour de l'axe des picots 271. La forme de la coque 3 est par ailleurs choisie de manière à ne pas empêcher l'introduction du cathéter 4 lorsque le système de blocage 1 est ouvert, c'est-à-dire lorsque la coque 3 n'est pas rabattue sur l'embase 2. Par exemple, la coque 3 peut être formée de sorte que son extrémité proximale vienne en butée contre la paroi externe de la bague 22 lorsque le système de blocage 1 est ouvert.

Le canal 23 quant à lui comprend une ouverture s'étendant en regard de la coque 3 du système de blocage 1. Par exemple, le canal 23 peut présenter une paroi 232 en U, de section semi-circulaire, dans le prolongement de la bague 22, l'ouverture 231 s'étendant sur toute sa longueur. En variante le canal 23 peut être de forme tubulaire, l'ouverture 231 étant alors réalisée sous la forme d'une fenêtre de dimensions appropriées pour recevoir l'organe d'entraînement 33 de la coque 3.

L'actionneur 25 est adapté pour venir en contact avec le manchon 24 et le pousser en direction de la paroi distale 212 du logement 20 afin de réduire la section interne de la lumière de blocage 241 et de bloquer le cathéter 4 en position par rapport à l'embase 2.

A cet effet, l'actionneur 25 est mobile en translation le long de l'axe longitudinal X, et présente une partie proximale 252 de guidage, logée au moins partiellement dans la bague 22, et une partie distale 254 formant poussoir, logée au moins partiellement dans le canal 23.

Sa partie distale 254 est adaptée pour pousser le manchon 24, tandis que sa partie proximale 252 qui sert de guide permet de positionner le cathéter 4 par rapport au manchon 24 et à l'embase 2, et l'actionneur 25 dans la chambre de blocage 21.

L'actionneur 25 comprend une lumière de guidage 251 traversante destinée à recevoir le cathéter 4, dont l'extrémité proximale 253 peut être évasée afin de faciliter l'introduction du cathéter 4. Le diamètre interne de cette lumière de guidage 251 est choisi de manière à être légèrement plus grand que celui du cathéter 4, afin de permettre l'introduction du cathéter 4 dans la lumière 251 et faciliter ensuite son guidage vers le manchon 24. Par exemple, le diamètre de la lumière de guidage 251 peut être décroissant entre son extrémité proximale 253 évasée et son extrémité distale, disposée en regard de la lumière de blocage 241 du manchon. Selon une forme de réalisation, le diamètre de l'extrémité distale de la lumière de guidage 251 est au plus égal au diamètre de l'extrémité proximale de la lumière de blocage 241, afin d'éviter que le cathéter 4 ne bute contre le manchon 24 lors de son introduction.

La lumière de guidage 251 est de préférence coaxiale à l'actionneur 25. Ainsi, une fois le manchon 24 positionné dans la bague 22, la lumière de guidage 251 est coaxiale à la bague 22 (et donc au logement 20).

La forme de la partie proximale 252 de guidage de l'actionneur 25 est de préférence globalement complémentaire de celle de la bague 22, ce qui permet de garantir l'alignement de l'actionneur 25 avec canal 23 et la chambre de blocage 21. Ainsi, pour une bague 22 de forme globalement tubulaire, la partie de guidage 252 est de forme globalement cylindrique de révolution. Le diamètre de la partie de guidage 252 est par ailleurs choisi en fonction du diamètre interne de la bague 22, de manière à permettre l'insertion du guide dans la bague 22, et son maintien dans la direction de translation de l'actionneur 25.

La partie proximale 252 de guidage peut en outre comprendre des moyens 258 prévus pour empêcher l'actionneur 25 de sortir du système 1. Ces moyens 258 peuvent notamment être une ou plusieurs protubérances faisant saillie de la surface externe de la partie proximale 252, à distance de son extrémité proximale. Les dimensions de la protubérance 258 sont choisies de sorte que le diamètre externe de la partie proximale 252 au niveau de la protubérance 258 soit plus grand que le diamètre interne de la bague 22. De la sorte, une fois l'actionneur 25 mis en place dans le système de blocage 1, celui-ci ne peut plus être extrait du logement 20 dans la mesure où la protubérance 258 l'empêche de se déplacer au-delà d'un certain point en direction de l'extrémité proximale de la bague 22.

De manière optionnelle, la position axiale de la protubérance 258 peut également être choisie de manière à garantir qu'un piston 257 de l'actionneur 25, tel qu'on le décrira dans la suite, soit toujours en contact avec le manchon 24, que l'actionneur 25 soit en position de repos, dans laquelle il ne déforme pas axialement le manchon 24, ou en position de blocage, dans laquelle l'actionneur 25 est introduit dans la chambre de blocage 21 afin de déformer le manchon 24. Ceci permet en effet de garantir la continuité des lumières de guidage 251 et de blocage 241, même en position de repos de l'actionneur 25, est donc de faciliter l'introduction d'un cathéter 4.

La partie distale 254 quant à elle s'étend globalement dans le canal 23, et comprend un corps 256, le piston 257 précité et l'organe d'entraînement 255. Le corps 256, le piston 257 et l'organe d'entraînement 255 peuvent notamment être monobloc. En variante, le piston 257 et l'organe d'entraînement 255 peuvent être rapportés sur le corps 256.

Le corps 256 s'étend le long de l'axe longitudinal X, entre la partie proximale 252 de guidage de l'actionneur 25 et l'extrémité distale de l'actionneur 25, et relie le piston 257 à l'organe d'entraînement 255. Le diamètre du corps 256 peut notamment être inférieur au diamètre interne de la bague 22 et/ou de la chambre de blocage 21.

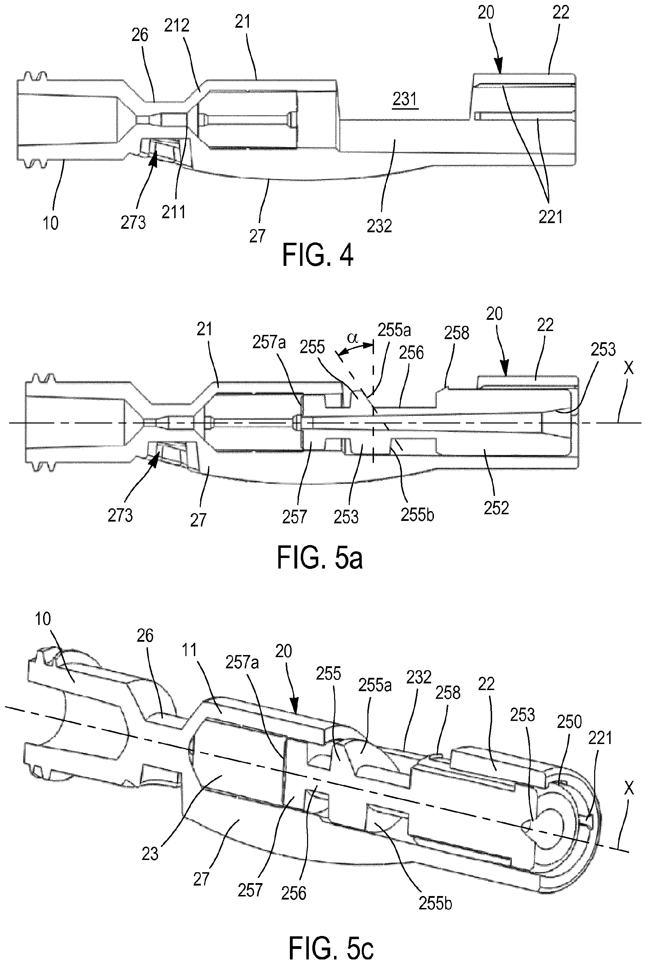

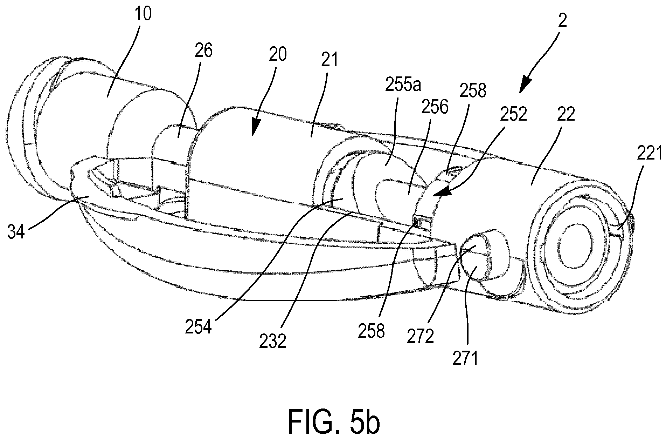

Le piston 257 est agencé au niveau de l'extrémité distale de la partie distale 254. Il a la forme d'une tête cylindrique, s'étendant transversalement par rapport à l'axe longitudinal X, et présente une paroi distale 257a sensiblement plane s'étendant transversalement à la direction de translation de l'actionneur 25. La tête peut par exemple être cylindrique de révolution, et avoir une épaisseur suffisante pour être capable de déformer le manchon 24 et bloquer un cathéter 4, comme illustré sur les figures 5b, 5c, 6b et 6c. En variante, la tête peut être conique et présenter un diamètre décroissant entre la paroi distale 257a du piston 257 et le corps 256.

L'organe d'entraînement 255 s'étend entre le piston 257 et la partie proximale 252 de guidage, et est agencé de manière à être disposé au moins partiellement en regard de l'ouverture 231 du canal 23. L'organe d'entraînement 255 est adapté pour coopérer avec l'organe d'entraînement 33 complémentaire de la coque 3. La forme des organes d'entraînement 255, 33 est déterminée de sorte que la fermeture de la coque 3 sur l'embase 2 entraine la translation de l'actionneur 25 en direction du manchon 24 sur une distance déterminée.

Par exemple, l'organe d'entraînement 255 de l'embase 2 présente une rampe proximale de renvoi d'efforts 255a engendrant un effort de poussée en direction du manchon 24 lorsqu'une force est appliquée sur ledit organe 255 dans une direction normale à la direction de translation. Pour cela, la rampe de renvoi 255 s'étend globalement transversalement au corps 256 de la partie distale 254, et la rampe est orientée en oblique avec un angle α compris préférentiellement entre 15° et 85° avec l'axe longitudinal X. L'angle α de la rampe de renvoi 255a est déterminé en fonction de la distance à parcourir par l'actionneur 25 pour comprimer axialement le manchon 24, et dépend du diamètre de l'organe 255. Ainsi, plus l'angle α sera petit, plus le diamètre de l'organe 255 devra être grand pour une même distance à parcourir.

L'effort de poussée est appliqué par l'organe d'entraînement 33 de la coque 3. Pour cela, l'organe d'entraînement 33 de la coque 3 a la forme d'une partie en saillie, adaptée pour coopérer avec la rampe de renvoi 255a afin de déplacer l'actionneur 25. Elle est agencée dans la coque 3 de manière à venir en contact avec l'organe 255 lorsque la coque 3 est rabattue sur l'embase 2 pour fermer le système de blocage 1, par exemple par rotation de la coque 3 par rapport à l'axe des picots 271, puis à prendre appui sur la rampe de renvoi 255a. L'actionneur 25 étant mobile en translation dans le logement 20, il est alors déplacé dans le logement 20 en direction du manchon 24 au fur et à mesure que la saillie progresse sur la rampe 255a, jusqu'au moment où la coque 3 se trouve en butée contre l'embase 2.

Dans l'exemple de réalisation illustré sur les figures, l'organe d'entraînement 255 présente une forme annulaire s'étendant autour du corps 256 de la partie distale 254 en direction de la coque 3, dont le diamètre externe est sensiblement égal à celui du manchon 24, et dont la rampe de renvoi 255a s'étend de part et d'autre du corps 256. La coque 3 peut alors avoir deux saillies 33, chacune étant adaptée pour venir en contact avec la rampe de renvoi 255a.

La coque 3 peut alors être verrouillée dans cette position à l'aide des moyens d'accrochage 32.

De manière optionnelle, la coque 3 peut également être maintenue en position par la coopération de l'organe 255 avec la saillie 33. Pour cela, l'organe 255 présente, dans le prolongement de la rampe 255a, en direction de la base 27 de l'embase 2, une paroi de verrouillage 255b s'étendant transversalement à l'axe longitudinal X. De la sorte, après avoir parcouru la rampe de renvoi 255a inclinée, la saillie 33 vient se placer en butée contre la paroi de verrouillage 255b de l'organe 255, ce qui verrouille le système 1 en position fermée. En effet, étant donné que le manchon 24 est déformé par l'actionneur 25 lorsque le système 1 est fermé, il applique un effort axial sur le piston 257 en direction de l'extrémité proximale du système de blocage 1 afin de reprendre sa forme initiale, ce qui plaque la paroi de verrouillage 255b contre la saillie 33. Le verrouillage peut être amélioré en utilisant une saillie 33 dont la paroi distale 33b est parallèle à la paroi de verrouillage 255b de l'organe 255 (lorsque le système de blocage 1 est fermé), de sorte que la paroi de verrouillage 255b et la paroi distale 33b se trouvent en contact sur toute leur surface, selon un plan perpendiculaire à la direction de l'effort appliqué par le manchon 24 (direction axiale, parallèle à l'axe longitudinal X). Le cas échéant, la paroi distale 33b de la saillie 33 peut en outre présenter une partie inclinée 33a complémentaire de la rampe de renvoi 255a, de sorte que lorsque le système 1 est fermé, la paroi distale 33b soit en contact à la fois avec la paroi de verrouillage 255b et la rampe de renvoi 255a, afin de renforcer le blocage de la coque 3 sur l'embase 2.

De manière avantageuse, le blocage de la coque 3 par coopération de la paroi de verrouillage 255b de l'organe 255 avec la saillie 33 crée une résistance supplémentaire lors du déverrouillage des crochets 322.

En variante, et de manière équivalente, l'actionneur 25 peut porter la saillie et la coque 3 l'organe à rampe de renvoi. Le fonctionnement du système de blocage 1 reste alors le même, la fermeture du système 1 entrainant le déplacement de l'actionneur 25 dans le logement en raison du glissement de la saillie (formant l'organe d'entraînement 255) sur la paroi d'entraînement de la came (formant l'organe d'entraînement 33 de la coque).

Dans le but de faciliter l'introduction d'un cathéter 4 dans la lumière de guidage 251, la longueur de la partie de guidage 252 peut être supérieure à la longueur de la bague 22, le long de l'axe longitudinal X. De la sorte, lorsque la partie proximale 252 de guidage se trouve en butée contre la protubérance 258, son extrémité proximale 253 fait saillie en dehors de la bague 22 et de l'embase 2, dégageant ainsi l'évasement de la lumière de guidage 251. Cette forme de réalisation offre par ailleurs l'avantage de permettre à un opérateur de vérifier que le système de blocage 1 n'est pas engagé et que le manchon 24 n'est pas comprimé, l'actionneur 25 étant visiblement dans sa position de repos.

Par ailleurs, afin d'éviter que l'actionneur 25 ne tourne sur lui-même, soit autour de l'axe longitudinal X, ce qui pourrait avoir pour conséquences que l'organe 255 ne se trouve plus en regard de la saillie 33 et empêcherait le blocage du cathéter 4, l'actionneur 25 et le logement peuvent être munis d'une ou plusieurs nervures et gorges rectilignes, s'étendant parallèlement à l'axe longitudinal X. Par exemple, la bague 22 peut comprendre quatre gorges 221 réparties de manière équidistante sur sa surface interne, coopérant avec autant de nervures 250 faisant saillie de la partie proximale 252 de guidage de l'actionneur, ou inversement.

En variante, les nervures 221, peuvent s'étendre depuis la partie distale 252 de l'actionneur 25, par exemple au niveau du piston 257, et coopérer avec et de gorges rectilignes 250 réalisées dans la chambre de blocage 21 du logement 20 (ou inversement). Dans cette variante de réalisation, la protubérance 258 est alors positionnée sur l'actionneur 25 de manière à garantir que le piston 257 de l'actionneur 25 reste dans la chambre de blocage 21 dans sa position de repos comme dans sa position de blocage.

Un opérateur peut donc connecter un cathéter 4, même de très petit diamètre, de manière simple, ergonomique, sûre, rapide et avec de faibles efforts, grâce à un système de blocage amovible 1 conforme à l'invention.

Pour cela, il ouvre tout d'abord le système de blocage 1, en écartant la coque 3 de l'embase 2 par rotation de la coque 3 autour de l'axe des picots 271. L'actionneur 25 n'exerce alors pas d'effort sur le manchon 24, dont la lumière de blocage 241 est par conséquent ouverte afin de permettre l'introduction du cathéter 4, et son extrémité proximale 253 fait saillie le cas échéant en dehors de la bague 22. Il introduit ensuite le cathéter 4 dans la lumière de guidage 251 de l'actionneur 25, puis pousse sur le cathéter 4 afin de lui faire traverser successivement l'actionneur 25 et le manchon 24, jusqu'à atteindre la chambre de visualisation 26. Grâce à la chambre de visualisation 26, l'opérateur peut vérifier visuellement que l'extrémité distale du cathéter 4 est suffisamment proche de l'organe de connexion 10. Le cathéter 4 est alors prêt à être bloqué de manière amovible par le système de blocage 1, et s'étend à travers la lumière 251 de l'actionneur 25, la lumière 241 du manchon 24, et dans la chambre de visualisation 26.

L'opérateur peut ensuite rabattre la coque 3 sur l'embase 2, afin de fermer le système de blocage 1 et bloquer le cathéter 4. Lors de la fermeture, la saillie 33 de la coque 3 vient en butée contre la came 255 de l'actionneur 25. En poursuivant la fermeture malgré cette première résistance, l'actionneur 25 est déplacé en direction du manchon 24 qu'il déforme axialement (le manchon 24 étant retenu par la paroi distale 212 de la chambre de blocage 21). Cette déformation axiale, le long de l'axe longitudinal X de l'embase 2, a pour effet de réduire la section de la lumière de blocage 241 du manchon 24, et de bloquer le cathéter 4 en position dans ledit manchon 24.

L'opérateur peut alors fixer un dispositif médical, par exemple un filtre, en vissant un embout adapté sur le connecteur 10 du système 1.

Lorsque l'opérateur souhaite sortir le cathéter 4, il lui suffit de déconnecter le dispositif médical du connecteur 10, puis d'ouvrir le système de blocage 1, en insérant un objet de dimensions appropriées dans l'orifice 273 de l'embase 2, puis d'écarter la coque 3 de l'embase par rotation autour des picots 271 de l'embase 2 afin de désengager les éléments de blocage 255 et 33 et de ramener l'actionneur 25 dans sa position de repos. Le manchon 24 peut alors reprendre sa forme initiale et libérer le cathéter 4, permettant ainsi à l'opérateur d'extraire le cathéter du système de blocage 1.

## Revendications

1. Système de blocage amovible (1) d'un cathéter (4), notamment un cathéter d'anesthésie locorégionale, comprenant une embase (2) adaptée pour recevoir un cathéter (4), et une coque (3), montée à rotation sur l'embase (2) entre une position ouverte, dans laquelle la coque (3) est éloignée de l'embase (2), et une position fermée, dans laquelle la coque (3) est accrochée sur l'embase (2) et bloque le cathéter (4) en position, le système (1) étant **caractérisé en ce que** :
- l'embase (2) définit un logement (20) présentant une direction principale selon un axe longitudinal (X), ledit logement (20) abritant un manchon (24) pourvu d'une lumière de blocage (241) s'étendant le long de l'axe longitudinal (X) et adapté pour venir en butée contre une paroi distale (212) du logement (20),
- le système comprend un actionneur (25) pourvu d'une lumière de guidage (251) s'étendant le long de l'axe longitudinal (X) et mobile en translation le long de l'axe longitudinal (X) dans le logement (20) entre une position de repos et une position de blocage dans laquelle l'actionneur (25) applique un effort axial sur le manchon (24) afin de le déformer, l'actionneur (25) comprenant un premier organe d'entraînement (255),
- la lumière de blocage (241) et la lumière de guidage (251) sont adaptées pour recevoir un cathéter (4), et
- la coque (3) comprend un deuxième organe d'entraînement (33), adapté pour coopérer avec le premier organe d'entraînement (255) de l'actionneur (25) lorsque la coque (3) est déplacée de sa position ouverte vers sa position fermée, et déplacer l'actionneur (25) le long de la direction longitudinale (X) vers sa position de blocage.

2. Système de blocage amovible (1) selon la revendication 1, dans lequel le logement (20) comprend :
- une chambre de blocage (21),
- une bague (22), s'étendant dans le prolongement de la chambre de blocage (21), le long de l'axe longitudinal (X), et
- un canal (23), s'étendant entre la chambre de blocage (21) et la bague (22), le long de l'axe longitudinal (X).

3. Système de blocage amovible (1) selon l'une des revendications 1 et 2, dans lequel un diamètre externe de la partie distale (254) de l'actionneur (25) est au plus égal au diamètre interne de la bague (22).

4. Système de blocage amovible (1) selon l'une des revendications 1 à 3, dans lequel un diamètre interne de la lumière de blocage (241) du manchon (24) est sensiblement égal au diamètre externe du cathéter (4).

5. Système de blocage amovible (1) selon l'une des revendications 1 à 4, dans lequel le logement comprend une ouverture (231) s'étendant en regard du premier organe d'entraînement (255) de l'actionneur (25), le deuxième organe d'entraînement (33) de la coque (3) étant positionné de manière à coopérer avec le premier organe d'entraînement (255) à travers ladite ouverture (231) du logement (20).

6. Système de blocage amovible (1) selon l'une des revendications 1 à 5, dans lequel le premier organe d'entraînement (255) possède une rampe de renvoi (255a) s'étendant vers l'extérieur depuis la partie distale (254) de l'actionneur (25), et le deuxième organe d'entraînement (33) comprend une partie en saillie s'étendant depuis la coque (3), agencée de manière à venir en contact avec ladite rampe de renvoi (255) lorsque la coque (3) est amenée vers sa position fermée.

7. Système de blocage amovible (1) selon la revendication 6, dans lequel l'organe (255) est de forme globalement annulaire, et la rampe de renvoi (255a) est orientée en oblique par rapport à un plan normal à la direction longitudinale (X).

8. Système de blocage amovible (1) selon la revendication 7, dans lequel l'organe (255) comprend en outre une paroi proximale de verrouillage (255b) s'étendant depuis la rampe de renvoi (255a) dans un plan sensiblement perpendiculaire à la direction longitudinale (X), adaptée pour venir en contact avec une paroi distale (33b) de la saillie (33) lorsque la coque (3) est dans sa position fermée.

9. Système de blocage amovible (1) selon l'une des revendications 1 à 8, dans lequel la partie distale (254) comprend en outre une protubérance (258) s'étendant radialement depuis ladite partie distale (254), adaptée pour empêcher l'actionneur (25) de sortir du logement (20).

10. Système de blocage amovible (1) selon l'une des revendications 1 à 9, dans lequel l'actionneur (25) et le logement (20) sont munis d'une ou plusieurs nervures et gorges rectilignes (250, 221), s'étendant radialement depuis l'actionneur (25), adaptées pour empêcher la rotation de l'actionneur (25) dans l'embase (2).

11. Système de blocage amovible (1) selon l'une des revendications 1 à 10, dans lequel l'embase (2) comprend en outre un organe de connexion (10) à un dispositif médical, ledit organe de connexion (10) étant en communication fluidique avec le logement (20).

12. Système de blocage amovible (1) selon la revendication 11, comprenant en outre une chambre transparente de visualisation (26) s'étendant entre le logement (20) et l'organe de connexion (10), adaptée pour recevoir une extrémité libre du cathéter (4).

13. Système de blocage amovible (1) selon la revendication 12, dans lequel la coque (3) comprend des moyens d'accrochage (32, 322) à l'embase (2).

14. Système de blocage amovible (1) selon la revendication 13, dans lequel les moyens d'accrochage comprennent au moins un crochet (322) engagé dans un orifice (273) dans l'embase (2) de manière à pouvoir être libéré pour ouvrir le système de blocage amovible (1).

15. Système de blocage amovible (1) selon la revendication 13, dans lequel les moyens d'accrochage (32) comprennent des crochets (326), fixés sur la coque (3) et adaptés pour coopérer par encliquetage avec des languettes (325), s'étendant depuis l'embase (2), lesdites languettes étant élastiquement déformables de manière à permettre à un opérateur de libérer les crochets (326) par simple pression.

## Patentansprüche

1. Abnehmbares Blockierungssystem (1) eines Katheters (4), insbesondere eines Katheters für Regionalanästhesie, das einen Sockel (2) umfasst, der angepasst ist, um einen Katheter (4) aufzunehmen, und eine Schale (3), die drehend auf dem Sockel (2) zwischen einer offenen Position, in der die Schale (3) von dem Sockel (2) entfernt ist, und einer geschlossenen Position, in der die Schale (3) auf dem Sockel (2) angehängt ist und den Katheter (4) in Position blockiert, montiert ist, wobei das System (1) **dadurch gekennzeichnet ist, dass**:
- der Sockel (2) eine Aufnahme (20) definiert, die eine Hauptrichtung entlang einer Längsachse (X) aufweist, wobei die Aufnahme (20) einen Stutzen (24) unterbringt, der mit einem Blockierungslangloch (241) versehen ist, das sich entlang der Längsachse (X) erstreckt und angepasst ist, um gegen eine distale Wand (212) der Aufnahme (20) zum Anschlagen zu kommen,
- das System einen Aktuator (25) umfasst, der mit einem Führungslangloch (251) versehen ist, das sich entlang der Längsachse (X) erstreckt und in Verschiebung entlang der Längsachse (X) in der Aufnahme (20) zwischen einer Ruheposition und einer Blockierungsposition, in der der Aktuator (25) eine axiale Kraft auf den Stutzen (24) anlegt, um ihn zu verformen, beweglich ist, wobei der Aktuator (25) ein erstes Antriebsorgan (255) umfasst,
- das Blockierungslangloch (241) und das Führungslangloch (251) angepasst sind, um einen Katheter (4) aufzunehmen, und
- die Schale (3) ein zweites Antriebsorgan (33) umfasst, das angepasst ist, um mit dem ersten Antriebsorgan (255) des Aktuators (25) zusammenzuwirken, wenn die Schale (3) von ihrer offenen Position zu ihrer geschlossenen Position bewegt wird, um den Aktuator (25) entlang der Längsrichtung (X) zu seiner Blockierungsposition zu bewegen.

2. Abnehmbares Blockierungssystem (1) nach Anspruch 1, wobei die Aufnahme (20) Folgendes umfasst:
- eine Blockierungskammer (21),
- einen Ring (22), der sich in der Verlängerung der Blockierungskammer (21) entlang der Längsachse (X) erstreckt, und
- einen Kanal (23), der sich zwischen der Blockierungskammer (21) und dem Ring (22) entlang der Längsachse (X) erstreckt.

3. Abnehmbares Blockierungssystem (1) nach einem der Ansprüche 1 und 2, wobei ein Außendurchmesser des distalen Teils (254) des Aktuators (25) mindestens gleich dem Innendurchmesser des Rings (22) ist.

4. Abnehmbares Blockierungssystem (1) nach einem der Ansprüche 1 bis 3, wobei ein Innendurchmesser des Blockierungslanglochs (241) des Stutzens (24) im Wesentlichen gleich dem Außendurchmesser des Katheters (4) ist.

5. Abnehmbares Blockierungssystem (1) nach einem der Ansprüche 1 bis 4, wobei die Aufnahme eine Öffnung (231) umfasst, die sich gegenüber dem ersten Antriebsorgan (255) des Aktuators (25) erstreckt, wobei das zweite Antriebsorgan (33) der Schale (3) derart positioniert ist, dass es mit dem ersten Antriebsorgan (255) durch die Öffnung (231) der Aufnahme (20) zusammenwirkt.

6. Abnehmbares Blockierungssystem (1) nach einem der Ansprüche 1 bis 5, wobei das erste Antriebsorgan (255) eine Umlenkrampe (255a) besitzt, die sich ausgehend von dem distalen Teil (254) des Aktuators (25) nach außen erstreckt, und das zweite Antriebsorgan (33) einen vorstehenden Teil umfasst, der sich von der Schale (3) erstreckt, der derart eingerichtet ist, dass er mit der Umlenkrampe (255) in Berührung kommt, wenn die Schale (3) zu ihrer geschlossenen Position gebracht wird.

7. Abnehmbares Blockierungssystem (1) nach Anspruch 6, wobei das Organ (255) im Allgemeinen Ringform hat, und die Umlenkrampe (255a) schräg in Bezug zu einer Ebene senkrecht zu der Längsrichtung (X) ausgerichtet ist.

8. Abnehmbares Blockierungssystem (1) nach Anspruch 7, wobei das Organ (255) außerdem eine proximale Verriegelungswand (255b) umfasst, die sich von der Umlenkrampe (255a) in einer Ebene im Wesentlichen senkrecht zu der Längsrichtung (X) erstreckt, die angepasst ist, um mit einer distalen Wand (33b) des Vorsprungs (33) in Berührung zu kommen, wenn die Schale (3) in ihrer geschlossenen Position ist.

9. Abnehmbares Blockierungssystem (1) nach einem der Ansprüche 1 bis 8, wobei der distale Teil (254) außerdem einen Vorsprung (258) umfasst, der sich radial von dem distalen Teil (254) erstreckt, der angepasst ist, um den Aktuator (25) daran zu hindern, aus der Aufnahme (20) auszutreten.

10. Abnehmbares Blockierungssystem (1) nach einem der Ansprüche 1 bis 9, wobei der Aktuator (25) und die Aufnahme (20) mit einer oder mehr geradlinigen Rippen und Hohlkehlen (250, 221) versehen sind, die sich radial von dem Aktuator (25) erstrecken, die angepasst sind, um die Drehung des Aktuators (25) in dem Sockel (2) zu verhindern.

11. Abnehmbares Blockierungssystem (1) nach einem der Ansprüche 1 bis 10, wobei der Sockel (2) außerdem ein Organ zum Anschließen (10) an eine medizinische Vorrichtung umfasst, wobei das Anschlussorgan (10) in fluidtechnischer Kommunikation mit der Aufnahme (20) steht.

12. Abnehmbares Blockierungssystem (1) nach Anspruch 11, das außerdem eine durchsichtige Sichtkammer (26) umfasst, die sich zwischen der Aufnahme (20) und dem Anschlussorgan (10) erstreckt, die angepasst ist, um ein freies Ende des Katheters (4) aufzunehmen.

13. Abnehmbares Blockierungssystem (1) nach Anspruch 12, wobei die Schale (3) Mittel zum Anhängen (32, 322) an dem Sockel (2) umfasst.

14. Abnehmbares Blockierungssystem (1) nach Anspruch 13, wobei die Mittel zum Anhängen mindestens einen Haken (322) umfassen, der in eine Öffnung (273) in dem Sockel (2) derart eingreift, dass er freigegeben werden kann, um das abnehmbare Blockierungssystem (1) zu öffnen.

15. Abnehmbares Blockierungssystem (1) nach Anspruch 13, wobei die Mittel zum Anhängen (32) Haken (326) umfassen, die auf der Schale (3) befestigt und geeignet sind, um durch Einrasten mit Laschen (325), die sich von dem Sockel (2) erstrecken, zusammenzuwirken, wobei die Laschen elastisch derart verformbar sind, dass es einem Bediener erlaubt wird, die Haken (326) durch einfachen Druck freizugeben.

## Claims

1. Removable locking system (1) of a catheter (4), particularly a locoregional anaesthetic catheter, comprising a base (2) suitable for receiving a catheter (4), and a shell (3), rotatably mounted on the base (2) between an open position, wherein the shell (3) is removed from the base (2), and a closed position, wherein the shell (3) is fastened to the base (2) and locks the catheter (4) in position, the system (1) being **characterised in that**:
- the base (2) defines a housing (20) having a main direction along a longitudinal axis (X), said housing (20) accommodating a sleeve (24) provided with a locking slot (241) extending along the longitudinal axis (X) and suitable for abutting against a distal wall (212) of the housing (20),
- the system comprises an actuator (25) provided with a guiding slot (251) extending along the longitudinal axis (X) and movable in translation along the longitudinal axis (X) in the housing (20) between an idle position and a locking position wherein the actuator (25) applies an axial load on the sleeve (24) so as to deform same, the actuator (25) comprising a first actuation member (255),
- the locking slot (241) and the guiding slot (251) are suitable for receiving a catheter (4), and
- the shell (3) comprises a second actuation member (33), suitable for engaging with the first actuation member (255) of the actuator (25) when the shell (3) is moved from the open position thereof to the closed position thereof, and moving the actuator (25) along the longitudinal axis (X) to the locking position thereof.

2. Removable locking system (1) according to claim 1, wherein the housing (20) comprises:
- a locking chamber (21),
- a ring (22), extending along from the locking chamber (21), along the longitudinal axis (X), and
- a channel (23), extending between the blocking chamber (21) and the ring (22), along the longitudinal axis (X).

3. Removable locking system (1) according to any one of claims 1 and 2, wherein an outer diameter of the distal portion (254) of the actuator (25) is at most equal to the inner diameter of the ring (22).

4. Removable locking system (1) according to any one of claims 1 to 3, wherein an inner diameter of the locking slot (241) of the sleeve (24) is substantially equal to the outer diameter of the catheter (4).

5. Removable locking system (1) according to any one of claims 1 to 4, wherein the housing comprises an opening (231) extending facing the first actuation member (255) of the actuator (25), the second actuation member (33) of the shell (3) being positioned so as to engage with the first actuation member (255) via said opening (231) of the housing (20).

6. Removable locking system (1) according to any one of claims 1 to 5, wherein the first actuation member (255) has a return ramp (255a) extending outwards from the distal portion (254) of the actuator (25), and the second actuation member (33) comprises a protruding portion extending from the shell (3) arranged so as to come into contact with said return ramp (255) when the shell (3) is moved to the closed position thereof.

7. Removable locking system (1) according to claim 6, wherein the member (255) has an overall annular shape, and the return ramp (255a) is oriented obliquely with respect to a normal plane to the longitudinal direction (X).

8. Removable locking system (1) according to claim 7, wherein the member (255) further comprises a proximal locking wall (255b) extending from the return ramp (255a) in a plane substantially perpendicular to the longitudinal axis (X), suitable for coming into contact with a distal wall (33b) of the protrusion (33) when the shell (3) is in the closed position thereof.

9. Removable locking system (1) according to any one of claims 1 to 8, wherein the distal portion (254) further comprises a protuberance (258) extending radially from said distal portion (254), suitable for preventing the actuator (25) from coming out of the housing (20).

10. Removable locking system (1) according to any one of claims 1 to 9, wherein the actuator (25) and the housing (20) are equipped with one or a plurality of rectilinear ribs and grooves (250, 221), extending radially from the actuator (25), suitable for preventing the rotation of the actuator (25) in the base (2).

11. Removable locking system (1) according to any one of claims 1 to 10, wherein the base (2) further comprises a connection member (10) to a medical device, said connection member (10) being in fluidic communication with the housing (20).

12. Removable locking system (1) according to claim 11, further comprising a transparent viewing chamber (26) extending between the housing (20) and the connection member (10), suitable for receiving a free end of the catheter (4).

13. Removable locking system (1) according to claim 12, wherein the shell (3) comprises fastening means (32. 322) to the base (2).

14. Removable locking system (1) according to claim 13, wherein the fastening means comprise at least one hook (322) inserted into an orifice (273) in the base (2) so as to be able to be released to obtain the removable locking system (1).

15. Removable locking system (1) according to claim 13, wherein the fastening means (32) comprise hooks (326), attached to the shell (3) and suitable for engaging by detent with tabs (325), extending from the base (2), said tabs being elastically deformable so as to enable an operator to release the hooks (326) by merely pressing.
